# EUROPEAN PATENT APPLICATION

(11) **EP 1 886 697 A1**
(43) Date of publication of application: **13.02.2008**
(21) Application number: 06731278.5
(22) Date of filing: 06.04.2006
(51) Int. Cl.: A61L 2/20, B01D 53/26

(54) **STERILIZING SYSTEM**

(30) Priority: 17.05.2005 JP 2005144135
(71) Applicant: DAIKIN INDUSTRIES, LTD., Osaka-shi, Osaka 530-8323 (JP)
(72) Inventor: MIYAUCHI, Satoru, DAIKIN APPLIED SYSTEMS CO. LTD, Tokyo 1080023 (JP)
(74) Representative: Goddar, Heinz J.
(86) International application number: PCT/JP2006/307330
(87) International publication number: WO 2006/123484

(57) **Abstract**

There are provided: an air compressor **(11);** a first passage **(12)** connected between the air compressor **(11)** and a treatment chamber **(2)** for introducing compressed air to the treatment chamber **(2);** a hydrogen peroxide solution container **(13)** which stores hydrogen peroxide solution; a second passage **(14)** connected between the hydrogen peroxide solution container **(13)** and the first passage **(12);** and a sterilizing gas supplying apparatus **(10)** including an ejector **(15)** for introducing the hydrogen peroxide solution to the first passage **(12)** from the hydrogen peroxide solution container **(13).** Compressed air including the hydrogen peroxide is filled in the treatment chamber **(2)** until the inside pressure of the treatment chamber **(2)** becomes higher than the air pressure for sterilizing the entire space of the treatment chamber **(2).** This allows the concentration of the hydrogen peroxide to be uniform in the treatment chamber **(2),** thereby attaining uniform and sufficient sterilizing power in the entire space of the treatment chamber **(2)** and suppressing an increase in cost of the system.

## Description

### Technical Field

The present invention relates to a sterilizing system for sterilizing a treatment chamber with the use of hydrogen peroxide.

### Background Art

As a sterilizing system of this kind, there have been conventionally proposed a system in which a pharmaceuticals treatment apparatus as a target to be sterilized is placed in an isolator composing a closed space as a treatment chamber and the treatment apparatus is sterilized in the isolator (see Patent Document 1, for example)

In the sterilizing system of Patent Document 1, an outdoor air supply circuit and a hydrogen peroxide supply circuit are connected to the isolator. The outdoor air supply circuit introduces outdoor air into the isolator with the use of an air supply fun. The hydrogen peroxide supply circuit includes a hydrogen peroxide gas generator for generating a hydrogen peroxide gas as a sterilizing agent, an intake port connected to an exhaust duct provided at the lower part of the isolator, and a gas supply port connected to an outdoor air introduction chamber provided in the upper part of the isolator.

In the above sterilizing system, after the outdoor air is introduced into the isolator through the outdoor air supply circuit, the hydrogen peroxide gas as a sterilizing agent generated in the hydrogen peroxide gas generator is supplied to the isolator. Thereafter, a gas containing the hydrogen peroxide in the isolator is returned for circulation to the outdoor air introduction chamber through the exhaust duct of the isolator by the hydrogen peroxide supply circuit.
Patent Document 1: Japanese Patent Application Laid Open Publication No. 2001-000514

### Problems that the Invention is to Solve

In the above conventional sterilizing system, however, the hydrogen peroxide gas is introduced to a space in the isolator only after the outdoor air is supplied to the space, inviting non-uniformity of concentration of the hydrogen peroxide in the space. This forms in the space a part where sufficient sterilizing power is exhibited and a part where insufficient sterilizing power is exhibited (generally called a dead spot), attaining non-uniform sterilizing power in the space of the isolator as a whole.

In order to make the concentration of the hydrogen peroxide to be uniform in the space of the isolator, the air in the space may be agitated with the use of a blower, which increases, however, the cost of the system.

Further, in order to generate the hydrogen peroxide gas, a heater for heating an aqueous solution of the hydrogen peroxide is provided in the hydrogen peroxide gas generator in general. Provision of the heater itself increases cost of a system using such a heater.

The present invention has been made in view of the foregoing and has its object of attaining, in a sterilizing system for treating the inside of a treatment chamber with the use of hydrogen peroxide, uniform and sufficient sterilizing power in the entire space of the treatment chamber and preventing an increase in cost of the system by making the concentration of the hydrogen peroxide to be uniform in the treatment chamber.

A first aspect of the present invention is directed to a sterilizing system for sterilizing the inside of a treatment chamber (2). Wherein, the sterilizing system is characterized by including: an air compressor (11); a first passage (12) connected between the air compressor (11) and the treatment chamber (2) for introducing compressed air to the treatment chamber (2); a hydrogen peroxide solution container (13) which stores hydrogen peroxide solution; a second passage (14) connected between the hydrogen peroxide solution container (13) and the first passage (12); and a sterilizing gas supplying apparatus (10) including a hydrogen peroxide solution introducing mechanism (15) for introducing the hydrogen peroxide solution to the first passage (12) from the hydrogen peroxide solution container (13).

In the first aspect of the present invention, compressed air is pushed into the treatment chamber (2) from the air compressor (11) through the first passage (12). When flowing in the first passage (12), the compressed air is mixed with the hydrogen peroxide from the hydrogen peroxide solution container (13) through the second passage (14). When the compressed air is mixed with the hydrogen peroxide, the hydrogen peroxide is atomized to disperse in the compressed air uniformly. When the compressed air (sterilizing gas) uniformly including the hydrogen peroxide is introduced into the treatment chamber (2), the hydrogen peroxide disperses uniformly in the treatment chamber (2).

Particularly, pushing the compressed gas into the treatment chamber (2) increases the inside pressure of the treatment chamber (2) higher than the air pressure to allow the concentration of the hydrogen peroxide to be uniform in the treatment chamber (2). As a result, uniform and sufficient sterilizing power can be attained in the entire space of the treatment chamber (2).

Referring to a second aspect of the present invention, in the first aspect, the hydrogen peroxide introducing mechanism (15) is composed of an ejector (15) including a drive passage (15a) through which high-pressure fluid flows and a suction passage (15b) joined to the drive passage (15a), and in the ejector (15), the drive passage (12a) is connected to the first passage (12) while the suction passage (15b) is connected to the second passage (14) at a part of the sterilizing gas supplying apparatus (10) where the first passage (12) and the second passage (14) are joined to each other.

In the second aspect, the high-pressure compressed air flows in the drive passage (15a) connected to the first passage (12) to generate negative pressure in the suction passage (15b), so that the hydrogen peroxide solution flows through the second passage (14) from the hydrogen peroxide solution container (13) to be sucked into the second passage (14). When it is sucked, the hydrogen peroxide solution is adiabatically expanded and is atomized to disperse uniformly in the compressed air.

Referring to a third aspect of the present invention, the sterilizing system in the first invention further includes: a dehumidifier (40) which dehumidifies air in the treatment chamber (2).

In the third aspect, the dehumidifier (40) dehumidifies the inside of the treatment chamber (2). It has been known that in a sterilizing system of this kind, the lower the humidity of air in the space of the treatment chamber (2) is, the higher the level of the sterilizing power becomes. Accordingly, when the treatment chamber (2) is filled with the compressed air including the hydrogen peroxide after or during dehumidification of the inside of the treatment chamber (2), high-level sterilizing effect can be obtained in the entire treatment chamber (2).

Referring to a fourth aspect of the present invention, in the third aspect, the dehumidifier (40) includes: a first air passage (41) through which treatment air for dehumidification flows; a second air passage (42) through which high-temperature air for regeneration flows; and an adsorption rotor (43) arranged across both the air passages (41, 42) and capable of rotating about a part between the first air passage (41) and the second air passage (42) as a center, and one end of the first air passage (41) is connected to the treatment chamber (2) so as to function as an air supply port (46) for supplying air from the adsorption rotor (43) to the treatment chamber (2) while the other end of the first air passage (41) is connected to the treatment chamber (2) so as to function as an air intake port (47) for sucking air from the treatment chamber (2) to the adsorption rotor (43).

In the fourth aspect, the treatment air for dehumidification flows in the first air passage (41), is dehumidified when passing through the adsorption rotor (43), and is then supplied to the treatment chamber (2) through the air supply port (46). The air in the treatment chamber (2) is sucked into the dehumidifier (40) through the air suction port (47), is dehumidified again in the adsorption rotor (43), and then, is supplied to the treatment chamber (2). On the other hand, the adsorption rotor (43) is rotated continuously or continually so that a part thereof which has adsorbed moisture moves toward the second air passage (42). In the second passage (14), when the high-temperature air for regeneration passes through the adsorption rotor (43), the moisture adsorbed in the adsorption rotor (43) is desorbed, thereby regenerating the adsorption rotor (43).

Referring to a fifth aspect of the present invention, in the third aspect, dehumidification of the inside of the treatment chamber (2) by the dehumidifier (40) and sterilizing gas supply to the treatment chamber (2) by the sterilizing gas supplying apparatus (10) are performed alternately.

In the fifth aspect, the dehumidification in the treatment chamber (2) and the sterilizing gas supply to the treatment chamber (2) are performed alternately to push the compressed air including the hydrogen peroxide into the entirety of the treatment chamber (2) with the humidity of the inside of the treatment chamber (2) kept low, thereby sterilizing the entire treatment chamber (2) uniformly.

Referring to a sixth aspect of the present invention, in the third aspect, dehumidification of the inside of the treatment chamber (2) by the dehumidifier (40) and sterilizing gas supply to the treatment chamber (2) by the sterilizing gas supplying apparatus (10) are performed simultaneously.

In the sixth aspect, the dehumidification in the treatment chamber (2) and the sterilizing gas supply to the treatment chamber (2) are performed simultaneously to push the compressed air including the hydrogen peroxide into the entirety of the treatment chamber (2) with the humidity of the inside of the treatment chamber (2) kept low, thereby sterilizing the entire treatment chamber (2) uniformly.

Referring to a seventh aspect of the present invention, in the first aspect, an air blow port (31) for blowing out air to the treatment chamber (2) and an air take port (32) for taking air from the treatment chamber (2) are formed in the treatment chamber (2), and the sterilizing system further includes: a circulation passage (33) connected to the air blow port (31) and the air take port (32) and being capable of circulating air outside the treatment chamber (2); and an air conditioner (30) incorporated in the circulation passage (33).

In the seventh aspect, the air conditioner (30) is incorporated in the circulation passage (33). Accordingly, the air conditioner (30) air-conditions the treatment chamber (2) during the time when the air taken out from the air take port **(32)** of the treatment chamber (2) flows through the circulation passage ***(33)** and is blown out into the treatment chamber (2) from the air blow port (31).

Referring to an eighth aspect of the present invention, in the seventh aspect, a blow port **(16)** of the first passage (12) of the sterilizing gas supplying apparatus **(10)** is connected at an upper part of the treatment chamber (2), and the air take port **(32)** of the circulation passage **(33)** is formed at a lower part of the treatment chamber **(2).**

In the eighth aspect, when the air conditioner **(30)** is operated with the sterilizing gas supplying apparatus **(10)** operated, the compressed gas including the hydrogen peroxide is blown from the upper part of the treatment chamber (2) to the space of the treatment chamber (2) while the air in the treatment chamber (2) is taken out from the air take port **(32)** formed at the lower part of the treatment chamber (2). This causes downward air flow from above in the treatment chamber (2) to permit the concentration distribution of the hydrogen peroxide to be uniform in the treatment chamber (2).

Referring to a ninth aspect of the present invention, in the seventh aspect, a blow port **(16)** of the first passage (12) of the sterilizing gas supplying apparatus (10) is connected at a lower par of the treatment chamber (2), and the air take port (32) of the circulation passage **(33)** is formed at an upper part of the treatment chamber (2).

In the ninth aspect, when the air conditioner **(30)** is operated with the sterilizing gas supplying apparatus **(10)** operated, the compressed gas including the hydrogen peroxide is blown from the lower part of the treatment chamber (2) to the space of the treatment chamber (2) while the air in the treatment chamber (2) is taken out from the air take port **(32)** formed at the upper part of the treatment chamber (2). This causes upward air flow from the lower part in the treatment chamber (2) to permit the concentration distribution of the hydrogen peroxide to be uniformed in the treatment chamber (2).

Referring to a tenth aspect of the present invention, a sterilizing system for sterilizing the inside of a treatment chamber (2) is characterized by including: a first passage **(12)** which forcedly sends compressed air to the air chamber (2); and a second passage **(12)** which mixes hydrogen peroxide solution with the compressed air in the first passage **(12)** so as to atomize the hydrogen peroxide in the compressed air in the first passage **(12),** wherein the compressed air including the hydrogen peroxide is filled in the treatment chamber **(2)** until the inside pressure of the treatment chamber (2) becomes higher than the air pressure to sterilize the entire treatment chamber **(2).**

In the tenth aspect, similarly to the first aspect of the present invention, pushing the compressed gas into the treatment chamber (2) increases the inside pressure of the treatment chamber (2) higher than the air pressure to allow the concentration of the hydrogen peroxide in the treatment chamber **(2)** to be uniform. As a result, uniform and sufficient sterilizing power can be attained in the entire space of the treatment chamber (2).

### Effects of the Invention

In the present invention, the sterilizing system includes: an air compressor **(11);** a first passage **(12)** connected between the air compressor **(11)** and the treatment chamber **(2)** for introducing compressed air to the treatment chamber **(2);** a hydrogen peroxide solution container **(13)** which stores hydrogen peroxide solution; a second passage **(14)** connected between the hydrogen peroxide solution container **(13)** and the first passage **(12);** and a sterilizing gas supplying apparatus **(10)** including a hydrogen peroxide solution introducing mechanism **(15)** for introducing the hydrogen peroxide solution to the first passage **(12)** from the hydrogen peroxide solution container **(13).** Accordingly, only by pushing the compressed gas including the hydrogen peroxide into the treatment chamber **(2)** until the chamber pressure becomes higher in pressure than the air pressure, uniform and sufficient sterilizing power can be attained in the entire space of the treatment chamber **(2).** Further, the present invention eliminates the need to provide a blower for agitating the inside air of the treatment chamber (2) and a heater for atomizing the hydrogen peroxide solution, thereby suppressing an increase in cost of the system.

In the second aspect of the present invention, the ejector **(15)** including the drive passage **(15a)** through which the high pressure fluid flows and the suction passage **(15b)** joined to the drive passage **(15a)** is employed as the hydrogen peroxide introducing mechanism **(15).** This simple mechanism attains atomization of the hydrogen peroxide in the compressed air to thus lead to suppression of an increase in cost of the system further definitely.

In the third aspect of the present invention, the dehumidifier **(40)** is provided for dehumidifying the air in the treatment chamber (2) to keep the humidity of the inside of the treatment chamber (2) low, thereby attaining further higher-level sterilizing effect in the entire treatment chamber (2).

In the fourth aspect of the present invention, the dehumidifier **(40),** which includes the low-cost and simple adsorption rotor **(43),** dehumidifies the inside of the treatment chamber (2) to attain high-level sterilizing effect in the entire treatment chamber (2).

According to the fifth aspect of the present invention, alternate operation of the dehumidification of the inside of the treatment chamber (2) by the dehumidifier (40) and the sterilizing gas supply to the treatment chamber (2) by the sterilizing gas supplying apparatus **(10)** causes the compressed air including the hydrogen peroxide to be pushed into the entirety of the treatment chamber (2) with the humidity of the inside of the treatment chamber (2) kept low, thereby sterilizing the entire treatment chamber (2).

According to the sixth aspect of the present invention, simultaneous operation of the dehumidification of the inside of the treatment chamber (2) by the dehumidifier **(40)** and the sterilizing gas supply to the treatment chamber (2) by the sterilizing gas supplying apparatus **(10)** causes the compressed air including the hydrogen peroxide to be pushed into entirety of the treatment chamber (2) with the humidity of the inside of the treatment chamber (2) kept low, thereby sterilizing the entire treatment chamber **(2).**

According to the seventh aspect of the present invention, air conditioning of the treatment chamber (2) can be performed by the air conditioner **(30)** incorporated in the circulation passage **(33)** during the time when the air taken out from the air take port **(32)** of the treatment chamber (2) flows through the circulation passage **(33)** and is blown out into the treatment chamber (2) from the air blow port **(31).**

In the eighth aspect of the present invention, the air blow port **(16)** of the first passage (12) of the sterilizing gas supplying apparatus (10) is connected at the upper part of the treatment chamber (2) while the air take port (32) of the circulation passage **(33)** is formed at the lower part of the treatment chamber (2). Accordingly, when the air conditioner (30) is operated with the sterilizing gas supplying apparatus **(10)** operated, the compressed gas including the hydrogen peroxide is blown down from the upper part of the treatment chamber (2) to the space of the treatment chamber (2) while the air in the treatment chamber (2) is taken out from the air take port **(32)** formed at the lower part of the treatment chamber (2). This causes downward air flow from the upper part in the treatment chamber (2), thereby permitting the concentration distribution of the hydrogen peroxide to be uniform in the treatment chamber (2) to thus achieve uniform sterilization in the entire treatment chamber **(2).**

In the ninth aspect of the present invention, the air blow port **(16)** of the first passage **(12)** of the sterilizing gas supplying apparatus **(10)** is connected at the lower part of the treatment chamber (2) while the air take port **(32)** of the circulation passage **(33)** is formed at the upper part of the treatment chamber (2). Accordingly, when the air conditioner **(30)** is operated with the sterilizing gas supplying apparatus **(10)** operated, the compressed gas including the hydrogen peroxide is blown up from the lower part of the treatment chamber (2) to the space of the treatment chamber (2) while the air in the treatment chamber (2) is take out from the air taking port (32) formed at the upper part of the treatment chamber (2). This causes upward air flow from the lower part in the treatment chamber (2), thereby permitting the concentration distribution of the hydrogen peroxide to be uniformed in the treatment chamber **(2)** to thus achieve uniform sterilization in the entire treatment chamber (2).

According to the tenth aspect of the present invention, only by pushing the compressed gas including the hydrogen peroxide into the treatment chamber (2) until the chamber pressuer becomes higher in pressure than the air pressure, uniform and sufficient sterilizing power can be attained in the entire space of the treatment chamber (2). Further, the present invention eliminates the need to provide a blower for agitating the inside air of the treatment chamber (2) and a heater for atomizing the hydrogen peroxide, thereby suppressing an increase in cost of the system.

### Brief Description of the Drawings

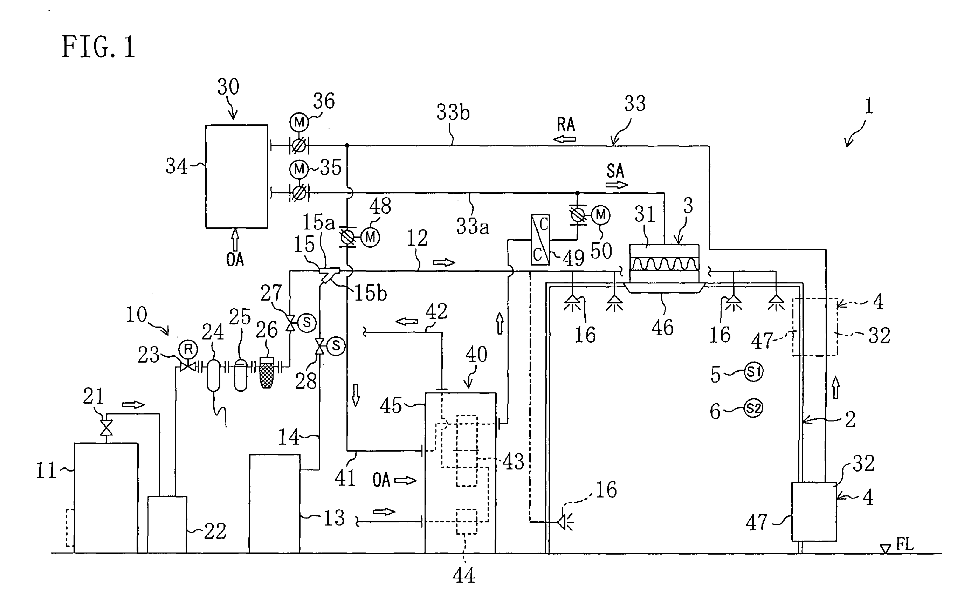

[FIG.1] FIG. **1** is a diagram showing a schematic construction of a sterilizing system in accordance with an embodiment of the present invention.

### Explanation of Reference Numerals

- **1**: sterilizing system
- **2**: treatment chamber
- **3**: air supply unit
- **4**: air return unit
- **10**: sterilizing gas supplying apparatus
- **11**: air compressor
- **12**: first passage
- **13**: hydrogen peroxide solution container
- **14**: second passage
- **15**: ejector (hydrogen peroxide solution introducing mechanism)
- **15a**: drive passage
- **15b**: suction passage
- **30**: air conditioner
- **31**: air blow port
- **32**: air take port
- **33**: circulation passage
- 40: dehumidifier
- **41**: first air passage
- **42**: second air passage
- **43**: adsorption rotor
- **46**: air supply port
- **47**: air intake port

### Best Mode For Carrying out the Invention

An embodiment of the present invention will be described below in detail with reference to the accompanying drawings.

As shown in FIG. **1****,** a sterilizing system (1) in accordance with the present embodiment serves as a system for sterilizing the inside as a to-be-sterilized space of a treatment chamber **(2)** (a clean room) and includes a sterilizing gas supplying apparatus **(10)** for supplying a sterilizing gas to the treatment chamber (2), an air conditioner **(30)** for air-conditioning the inside of the treatment chamber **(2),** and a dehumidifier **(40)** for dehumidifying air in the treatment chamber **(2).**

An air supply unit (3), which functions as an air supply port **(46)** of the dehumidifier **(40)** and an air blow port **(31)** of the air conditioner **(30),** is provided in the ceiling of the treatment chamber (2), and an air return unit **(4),** which functions as an air intake port **(47)** of the dehumidifier **(40)** and an air take port **(32)** of the air conditioner (30), is provided at the lower part of a side wall of the treatment chamber (2). The air supply unit **(3)** is integrated with an indoor unit **(31)** of the air conditioner **(30)** while on the other hand the air return unit **(4)** is integrated with a resolving device for resolving hydrogen peroxide. A pressure sensor (5) for detecting the inside pressure of the chamber and a concentration sensor **(6)** for detecting the concentration of the hydrogen peroxide in the chamber are provided in the inside of the treatment chamber (2).

The sterilizing gas supplying apparatus **(10)** includes an air compressor **(11),** a first passage **(12),** a hydrogen peroxide solution container **(13),** a second passage **(14),** and a hydrogen peroxide introducing mechanism **(15).** The first passage **(12)** is connected between the air compressor **(11)** and the treatment chamber **(2)** for introducing compressed air into the treatment chamber **(2).** The hydrogen peroxide solution container **(13)** stores aqueous solution of hydrogen peroxide. The second passage **(14)** is connected between the hydrogen peroxide solution container **(13)** and the first passage **(12).** The hydrogen peroxide introducing mechanism **(15)** introduces the hydrogen peroxide solution from the hydrogen peroxide solution container **(13)** to the first passage **(12).** The first passage **(12)** branches at the end part on the treatment chamber **(2)** side thereof into plural to be connected to the ceiling at the upper part of the treatment chamber **(2).** The end part on the treatment chamber **(2)** side of the first passage **(12)** branches into plural along the ceiling of the treatment chamber **(2)** in a crisscross pattern, and a splay nozzle (a splay port) **(16)** open to the inside of the treatment chamber **(2)** is provided at each end thereof.

The hydrogen peroxide introducing mechanism **(15)** is composed of an ejector **(15)** including a drive passage **(15a)** through which high-pressure fluid flows and a suction passage **(15b)** joined to the drive passage **(15a).** In the ejector **(15),** the drive passage **(15a)** is connected to the first passage **(12)** while the suction passage **(15b)** is connected to the second passage **(14)** at a part where the first passage **(12)** and the second passage **(14)** of the sterilizing gas supplying apparatus **(10)** are joined to each other.

In the first passage **(12)** between the air compressor **(11)** and the ejector **(15),** there are provided in this order from the upstream side to the downstream side of the air flow an opening/closing valve **(21),** an air dryer **(22),** an air regulator **(23),** an automatic drain **(24),** an oil mist separator **(25),** a high-performance filter **(26),** and a first automatic on/off valve **(27).** In the second passage **(14)** between the hydrogen peroxide solution container (13) and the ejector **(15),** a second automatic on/off valve (28) is provided.

The air conditioner **(30)** is composed of: an air supply unit (3) integrally formed with an indoor unit **(31)** and functioning as an air blow port **(31)** for blowing air into the treatment chamber **(2);** an air return unit **(4)** functioning as an air take port **(32)** for taking air out from the treatment chamber (2); a circulation passage **(33)** connected to the air blow port **(31)** and the air take port **(32)** and being capable of circulating air outside the treatment chamber (2); and an outdoor unit **(34)** provided in the circulation passage (33). In short, the air conditioner **(30)** is incorporated in the circulation passage **(33).**

The circulation passage **(33)** is composed of an air supply side passage (33a) and an air return side passage **(33b).** A first non-leakage automatic damper **(35)** is provide in the vicinity of a part of the air supply side passage **(33a)** which is connected to the outdoor unit **(34)** while a second non-leakage automatic damper (36) is provided in the vicinity of a part of the air return side passage **(33b)** which is connected to the outdoor unit **(34).**

The dehumidifier **(40)** includes: a first air passage **(41)** through which treatment air for dehumidification flows; a second air passage **(42)** through which high-temperature air for regeneration flows; and an adsorption rotor **(43)** arranged across both the air passages **(42, 42)** and being rotatable about a part between the first air passage **(41)** and the second air passage **(42)** as a center. The adsorption rotor **(43)** is accommodated in a casing **(45)** together with a heater **(44).** The regenerating air is heated at high temperature by receiving heat from the heater **(44)** provided upstream of the adsorption rotor **(43)** in the second air passage **(44).**

One end of the first air passage **(41)** is joined to the air supply side passage (33a), to be connected to the air supply port **(46)** (the air supply unit **(3))** that supplies air from the adsorption rotor **(43)** to the treatment chamber (2). The other end of the first air passage **(41)** is joined to the air return side passage **(33b)** to be connected to the air intake port **(47)** (the air return unit **(4))** that sucks air from the treatment chamber (2) to the adsorption rotor **(43).** A third non-leakage automatic damper **(48)** is provided in the first air passage **(41)** between the return unit **(4)** and the casing **(45).** In the first air passage **(41),** a cooling coil **(49)** and a fourth non-leakage automatic damper **(50)** are provided in this order from the upstream side to the downstream side of the air flow between the casing **(45)** and the air supply unit **(3).**

Each component of the sterilizing system **(1)** is made of a material having corrosion proof against hydrogen peroxide for preventing corrosion by the hydrogen peroxide, such as aluminum.

### -Driving operation-

Driving operations of the sterilizing system **(1)** will be described next.

### <Driving Operation Example 1>

Driving Operation Example 1 refers to a driving operation in which dehumidification of the inside of the treatment chamber (2) by the dehumidifier **(40)** and sterilizing gas supply to the treatment chamber (2) by the sterilizing gas supplying apparatus **(10)** are performed alternately.

In Driving Operation Example 1, the treatment chamber **(2)** (a clean room) is cleaned first. Then, a sterilizing operation starts. At start, the first non-leakage automatic damper (35) and the second non-leakage automatic damper **(36)** are closed while the third non-leakage automatic damper **(48)** and the fourth non-leakage automatic damper (50) are closed, and the dehumidifier **(40)** is activated.

In the dehumidifier **(40),** the adsorbent of the adsorption rotor (43) adsorbs moisture in the treatment air when the air passes through the adsorption rotor (43), so that the treatment air is dehumidified. The thus dehumidified treatment air flows through the first air passage **(41),** the cooling coil **(49),** and the air supply side passage **(33a)** to be supplied to the treatment chamber **(2)** through the air supply unit **(3)**. On the other hand, air including moisture in the treatment chamber **(2)** is sucked into the air return unit **(4),** flows through the air return side passage (33b) and the first air passage **(41),** returns to the casing **(45)** of the dehumidifier **(40),** and then passes through the adsorption rotor **(43)** again. Repetition of the above operation dehumidifies the inside of the treatment chamber (2).

A part of the adsorption rotor **(43)** where the treatment air of the first air passage (41) flows increases gradually in moisture adsorption amount to approximate to the saturated state. Since the adsorption rotor **(43)** is, however, rotated continuously or intermittently to move a part of the adsorption rotor **(43)** which has adsorbed moisture toward the second air passage **(42)** to allow high-temperature regenerating air heated in the heater **(44)** to flow in the second air passage **(42),** thereby performing regeneration (moisture desorption) of the part that has adsorbed the moisture. The air after regeneration by the adsorption rotor **(43)** is discharged outdoors. This attains continuous dehumidification of the inside of the treatment chamber **(2).**

When the dehumidification is continued for a predetermined time period or when it is detected that the humidity of the inside of the treatment chamber **(2)** lowers to a predetermined value, the dehumidification is stopped. Then, the third non-leakage automatic damper **(48)** and the fourth non-leakage automatic damper **(50)** are closed, and the air compressor **(11)** and the air dryer **(22)** are activated. At the same time, the first automatic on/off valve **(27)** and the second automatic on/off valve **(28)** are opened.

From the air compressor **(11),** the compressed air flows out, passes through the air dryer **(22)** to be dehumidified, is subjected to pressure adjustment by the air regulator **(23),** drain removal by the automatic drain, oil mist removal by the oil mist separator **(25),** and minute dust removal by the high-performance filter **(26),** and then, passes through the drive passage **(15a)** of the ejector **(15).** When the high-pressure compressed air flows through the drive passage **(15a)** of the ejector **(15),** negative pressure is generated in the suction passage **(15b)** to cause the liquid-state hydrogen peroxide in the hydrogen peroxide solution container to be sucked into the first passage (12). The hydrogen peroxide solution sucked in the first passage **(12)** is adiabatically expanded to be atomized. Then, the compressed air including the hydrogen peroxide is pushed into the treatment chamber (2) through the spray nozzles **(16).** Pushing of the compressed air into the treatment chamber (2) is continued until the pressure in the treatment chamber (2) and the concentration of the hydrogen peroxide reach respective predetermined values (for example, the pressure in the chamber reaches at the air pressure plus 50 Pa and the concentration of the hydrogen peroxide reaches 1000 ppm).

When the pressure in the treatment chamber **(2)** and the concentration of the hydrogen peroxide reach the respective predetermined values, the air compressor **(11)** and the air dryer **(22)** are stopped, and the first automatic on/off valve **(27)** and the second automatic on/off valve **(28)** are closed. Thereafter, the dehumidification of the inside of the treatment chamber (2) by the dehumidifier **(40)** and the sterilizing gas supply to the treatment chamber (2) by the sterilizing gas supplying apparatus **(10)** are performed alternately every two to three hours, for example.

In the sterilizing system **(1)** of this kind, it is generally known that the lower the humidity in the space of the treatment chamber **(2)** is, the higher the level of the sterilizing effect becomes. In view of this, when the hydrogen peroxide is filled with the inside of the treatment chamber **(2)** after the dehumidification of the inside of the treatment chamber **(2),** high-level sterilizing effect can be attained in the entire treatment chamber **(2).** Further, though the hydrogen peroxide filled therein will increase the humidity, repetition of the dehumidification and the hydrogen peroxide supply maintains the high-level sterilizing effect all the time.

It is noted that in normal operation of the air conditioner, the first non-leakage automatic damper **(35)** and the second non-leakage automatic damper (36) are opened so that the conditioned air is supplied indoors through the air supply unit (3) while the indoor air passes through the air return unit **(4)** to the circulation passage (33) and is returned to the outdoor unit **(34).** The outdoor unit (34) sends air to the air supply unit (3) while taking outdoor air.

### <Driving Operation Example 2>

Driving Operation Example 2 refers to a driving operation in which dehumidification of the inside of the treatment chamber (2) by the dehumidifier (40) and sterilizing gas supply to the treatment chamber (2) by the sterilizing gas supplying apparatus (10) are performed simultaneously.

In this example, the dehumidifier (40) always performs dehumidification of the inside of the treatment chamber **(2)** while at the same time the sterilizing gas supplying apparatus **(10)** performs sterilizing gas supply to the treatment chamber (2) intermittently. Specifically, the sterilizing gas supplying apparatus **(10)** repeats, with the dehumidifier **(40)** kept operated, an operation in which: when the inside pressure of the chamber and the concentration of the hydrogen peroxide reach respective predetermined values, the sterilizing gas supplying apparatus **(10)** is stopped; and when the inside pressure of the chamber and the concentration of the hydrogen peroxide reach respective predetermined lower limits, the sterilizing gas supplying apparatus **(10)** starts operating again.

Even in the above operation, as well, filling of the hydrogen peroxide into the treatment chamber (2) attains high-level sterilizing effect.

The sterilizing gas supply may be performed continuously in some cases.

### -Effects of Embodiment-

As described above, according to the present embodiment, when the compressed air including the hydrogen peroxide is only pushed into the treatment chamber (2) until the inside pressure of the treatment chamber (2) becomes higher than the air pressure, uniform and sufficient sterilizing power can be attained in the entire space of the treatment chamber (2). Further, neither a blower for agitating the inside air of the treatment chamber (2) nor a heater for atomizing the hydrogen peroxide solution is needed, thereby suppressing an increase in cost of the system.

Particularly, the sterilizing system **(1)** includes the air compressor **(11),** the first passage **(12)** connected between the air compressor **(11)** and the treatment chamber (2) for introducing the compressed air to the treatment chamber (2); the hydrogen peroxide solution container **(13)** for storing the hydrogen peroxide solution; the second passage **(14)** connected between the hydrogen peroxide solution container **(13)** and the first passage **(12);** and the sterilizing gas supplying apparatus **(10)** including the hydrogen peroxide solution introducing mechanism **(15)** for introducing the hydrogen peroxide solution to the first passage **(12)** from the hydrogen peroxide solution container **(13).** This simplifies the construction of the system, and existing air piping equipment around the treatment chamber (2) can be utilized, resulting in suppression of an increase in cost of the system.

Moreover, the ejector **(15)** including the drive passage **(15a)** through which the high-pressure fluid flows and the suction passage **(15b)** joined to the drive passage **(15a)** is employed as the hydrogen peroxide solution introducing mechanism **(15).** With this simple construction, the hydrogen peroxide solution can be atomized in the compressed air to suppress an increase in cost of the system further definitely.

The dehumidifier **(40)** is provided for dehumidifying the air in the treatment chamber (2) to thus keep the humidity of the inside of the treatment chamber (2) low. This attains further higher-level sterilizing effect in the entire treatment chamber (2). Further, the dehumidifier **(40)** includes the adsorption rotor **(43),** which is low in cost and has a simple construction, thereby suppressing an increase in cost of the system.

Furthermore, even when the dehumidification of the inside of the treatment chamber **(2)** by the dehumidifier **(40)** and the sterilizing gas supply to the treatment chamber **(2)** by the sterilizing gas supplying apparatus **(10)** are performed alternately as in Driving Operation Example 1 or simultaneously as in Driving Operation Example 2, the compressed air including the hydrogen peroxide can be pushed into the entirety of the treatment chamber **(2)** with the humidity of the inside of the treatment chamber (2) kept low, thereby sterilizing the entire treatment chamber (2) uniformly.

In the above embodiment, when the air conditioner **(30)** is operated with the sterilizing gas supplying apparatus **(10)** operated, the compressed gas including the hydrogen peroxide is blown down in the space of the treatment chamber (2) from the upper part of the treatment chamber **(2)** while the air in the treatment chamber (2) is taken out from the air take port **(32)** formed at the lower part thereof. This causes downward air flow from the upper part in the treatment chamber (2) to allow the concentration distribution of the hydrogen peroxide to be uniform in the treatment chamber (2), thereby performing uniform sterilization in the entire treatment chamber (2).

### <Other Embodiment>

The above embodiment may have the following construction.

In the above embodiment, the first passage **(12)** of the sterilizing gas supplying apparatus **(10)** is connected at the upper part of the treatment chamber **(2)** while the air return unit **(4)** as the air take port **(32)** of the circulation passage **(33)** is formed at the lower part of the treatment chamber **(2).** In reverse, as indicted by the imaginary lines, the first passage **(12)** of the sterilizing gas supplying apparatus **(10)** may be connected at a lower part of the treatment chamber **(2)** when the air take port **(32)** of the circulation passage **(33)** is formed at an upper part of the treatment chamber **(2),** for example.

In the above construction, when the air conditioner **(30)** is operated with the sterilizing gas supplying apparatus **(10)** operated, the compressed gas including the hydrogen peroxide is blown up in the space of the treatment chamber **(2)** from the lower part of the treatment chamber **(2)** while the air in the treatment chamber **(2)** is taken out from the air take port **(32)** formed at the upper part of the treatment chamber **(2).** This causes upward air flow from the lower part in the treatment chamber **(2)** to allow the concentration distribution of the hydrogen peroxide in the treatment chamber **(2)** to be uniform, thereby performing uniform sterilization in the entire treatment chamber **(2).**

The hydrogen peroxide, which has a specific gravity larger than that of the air, is liable to gather at the lower part in the treatment chamber **(2).** Nevertheless, the above construction achieves uniform concentration distribution of the hydrogen peroxide more effectively.

It is noted that the above embodiments are mere essentially preferred examples and do not intend to limit the scopes of the present invention, the applicable subjects, and use.

### Industrial Utilization

As describe above, the present invention is useful for sterilizing systems for sterilizing a treatment chamber with the use of hydrogen peroxide.

## Claims

1. A sterilizing system for sterilizing the inside of a treatment chamber (2) comprising:
an air compressor **(11);**
a first passage (12) connected between the air compressor (11) and the treatment chamber **(2)** for introducing compressed air to the treatment chamber (2);
a hydrogen peroxide solution container **(13)** which stores hydrogen peroxide solution;
a second passage **(14)** connected between the hydrogen peroxide solution container **(13)** and the first passage **(12);** and
a sterilizing gas supplying apparatus **(10)** including a hydrogen peroxide solution introducing mechanism **(15)** for introducing the hydrogen peroxide solution to the first passage **(12)** from the hydrogen peroxide solution container **(13).**

2. The sterilizing system of Claim 1,
wherein the hydrogen peroxide solution introducing mechanism **(15)** is composed of an ejector **(15)** including a drive passage **(15a)** through which high-pressure fluid flows and a suction passage **(15b)** joined to the drive passage **(15a),** and
in the ejector **(15),** the drive passage (12a) is connected to the first passage **(12)** while the suction passage **(15b)** is connected to the second passage **(14)** at a part of the sterilizing gas supplying apparatus **(10)** where the first passage **(12)** and the second passage **(14)** are joined to each other.

3. The sterilizing system of Claim 1, further comprising:
a dehumidifier **(40)** which dehumidifies air in the treatment chamber (2).

4. The sterilizing system of Claim 3,
wherein the dehumidifier **(40)** includes: a first air passage **(41)** through which treatment air for dehumidification flows; a second air passage **(42)** through which high-temperature air for regeneration flows; and an adsorption rotor **(43)** arranged across both the air passages **(41, 42)** and capable of rotating about a part between the first air passage **(41)** and the second air passage **(42)** as a center, and
one end of the first air passage **(41)** is connected to the treatment chamber **(2)** so as to function as an air supply port **(46)** for supplying air from the adsorption rotor **(43)** to the treatment chamber **(2)** while the other end of the first air passage **(41)** is connected to the treatment chamber **(2)** so as to function as an air intake port **(47)** for sucking air from the treatment chamber **(2)** to the adsorption rotor **(43).**

5. The sterilizing system of Claim 3,
wherein dehumidification of the inside of the treatment chamber **(2)** by the dehumidifier **(40)** and sterilizing gas supply to the treatment chamber **(2)** by the sterilizing gas supplying apparatus **(10)** are performed alternately.

6. The sterilizing system of Claim 3,
wherein dehumidification of the inside of the treatment chamber **(2)** by the dehumidifier **(40)** and sterilizing gas supply to the treatment chamber **(2)** by the sterilizing gas supplying apparatus **(10)** are performed simultaneously.

7. The sterilizing system of Claim 1,
wherein an air blow port **(31)** for blowing out air to the treatment chamber **(2)** and an air take port **(32)** for taking air from the treatment chamber **(2)** are formed in the treatment chamber **(2),** and
the sterilizing system further comprising:
a circulation passage **(33)** connected to the air blow port **(31)** and the air take port **(32)** and being capable of circulating air outside the treatment chamber **(2);** and
an air conditioner **(30)** incorporated in the circulation passage **(33).**

8. The sterilizing system of Claim 7,
wherein a blow port **(16)** of the first passage **(12)** of the sterilizing gas supplying apparatus **(10)** is connected at an upper part of the treatment chamber **(2),** and
the air take port **(32)** of the circulation passage **(33)** is formed at a lower part of the treatment chamber **(2).**

9. The sterilizing system of Claim 7,
wherein a blow port **(16)** of the first passage **(12)** of the sterilizing gas supplying apparatus (10) is connected at a lower part of the treatment chamber (2), and
the air take port **(32)** of the circulation passage **(33)** is formed at an upper part of the treatment chamber **(2).**

10. A sterilizing system for sterilizing the inside of a treatment chamber **(2)** comprising:
a first passage **(12)** which forcedly sends compressed air to the air chamber **(2);** and
a second passage **(12)** which mixes hydrogen peroxide with the compressed air in the first passage **(12)** so as to atomize the hydrogen peroxide in the compressed air in the first passage **(12),**
wherein the compressed air including the hydrogen peroxide is filled in the treatment chamber **(2)** until the inside pressure of the treatment chamber **(2)** becomes higher than the air pressure to sterilize the entire treatment chamber **(2).**
